# EUROPEAN PATENT APPLICATION

(11) **EP 4 400 158 A1**
(43) Date of publication of application: **17.07.2024**
(21) Application number: 21956456.4
(22) Date of filing: 14.09.2021
(51) Int. Cl.: A61N 1/36, A61B 5/383

(54) **METHOD AND SYSTEM FOR RECOMMENDING STIMULATION ELECTRODE COMBINATION**

(30) Priority: 09.09.2021 CN 202111054568
(71) Applicant: Sceneray Co., Ltd, Suzhou, Jiangsu 215123 (CN)
(72) Inventor: LIU, Bin, Suzhou, Jiangsu 215000 (CN); ZHU, Weiran, Suzhou, Jiangsu 215000 (CN)
(74) Representative: Cabinet Beaumont
(86) International application number: PCT/CN2021/118195
(87) International publication number: WO 2023/035279

(57) **Abstract**

Provided are a method and system for recommending a stimulation electrode combination. The method includes the following steps: Potentials of a plurality of electrodes are received in the process of treatment delivery; a difference between potentials of any two electrodes is calculated based on the potentials of the plurality of electrodes to obtain a voltage between any two electrodes; a feature signal corresponding to an electrode combination formed by any two electrodes is acquired based on the voltage between any two electrodes within a preset time range; and a recommended stimulation electrode combination is determined from all electrode combinations based on a feature signal corresponding to each electrode combination. The manner of simultaneous stimulation and collection can accurately reflect the real-time state of a patient when receiving electrical stimulation, improving the accuracy of the stimulation electrode combination. The arrangement of acquiring the feature signal according to the voltage between any two electrodes and determining the recommended stimulation electrode combination can reduce the workload of a doctor, improve the working efficiency of the doctor, and further improve the accuracy of the doctor in selecting a stimulation electrode combination.

## Description

### TECHNICAL FIELD

The present application relates to the field of implantable medical device technology and, in particular, to a method and system for recommending a stimulation electrode combination.

### BACKGROUND

Implantable medical systems generally include implantable nerve electrical stimulation systems, implantable cardiac electrical stimulation systems, and implantable drug delivery systems.

An implantable nerve electrical stimulation system is taken as an example. The implantable nerve electrical stimulation system mainly includes an implanted pulse generator, an electrode, and an extracorporeal control apparatus. The pulse generator is connected to the electrode. Therefore, a pulse generated by the pulse generator is transmitted to the electrode. A pulse signal generated by the pulse generator is transmitted through the electrode to a specific nerve for electrical stimulation so that the human body function can be restored to normal operation.

At present, the collection of a deep brain electrical signal of a patient is performed in the case where the patient is not stimulated. The collected signal cannot reflect the brain condition of the patient when being stimulated. Moreover, the selection of a stimulation electrode combination often depends on a doctor's experience, increasing the workload of the doctor and reducing the working efficiency of the doctor. Moreover, when the selection of the stimulation electrode combination only depends on manual work, the accuracy is not high enough.

### SUMMARY

An object of the present application is to provide a method and system for recommending a stimulation electrode combination to solve problems in the related art that a collected signal cannot reflect the brain condition of a patient when being stimulated; that the selection of a stimulation electrode combination often depends on a doctor's experience, increasing the workload of the doctor and reducing the working efficiency of the doctor; and that when the selection of the stimulation electrode combination only depends on manual work, the accuracy is not high enough.

The object of the present application is implemented through the technical solutions below.

In a first aspect, the present application provides a method for recommending a stimulation electrode combination. The method includes the following steps: Potentials of a plurality of electrodes are received in a process of treatment delivery; a difference between potentials of any two electrodes of the plurality of electrodes is calculated based on the potentials of the plurality of electrodes to obtain a voltage between the any two electrodes; a feature signal corresponding to an electrode combination formed by the any two electrodes is acquired based on the voltage between the any two electrodes within a preset time range; and a recommended stimulation electrode combination is determined from all electrode combinations based on a feature signal corresponding to each electrode combination.

In some optional embodiments, the step of determining the recommended stimulation electrode combination from the all electrode combinations based on the feature signal corresponding to the each electrode combination includes that a score corresponding to the each electrode combination is acquired based on one or more of a signal strength of the feature signal corresponding to the each electrode combination, a pulse width of the feature signal corresponding to the each electrode combination, or a similarity between the feature signal and a desired signal and that an electrode combination with a highest score is taken as the recommended stimulation electrode combination based on scores corresponding to the all electrode combinations.

In some optional embodiments, the step of acquiring, based on one or more of the signal strength of the feature signal corresponding to the each electrode combination, the pulse width of the feature signal corresponding to the each electrode combination, or the similarity between the feature signal and the desired signal, the score corresponding to the each electrode combination includes that a weight coefficient corresponding to the signal strength of the feature signal, a weight coefficient corresponding to the pulse width of the feature signal, and a weight coefficient corresponding to the similarity between the feature signal and the desired signal are configured separately, that a score of the signal strength of the feature signal corresponding to the each electrode combination, a score of the pulse width of the feature signal corresponding to the each electrode combination, and a score of the similarity between the feature signal and the desired signal are acquired separately, and that weighted summation on the score of the signal strength of the feature signal corresponding to the each electrode combination, the score of the pulse width of the feature signal corresponding to the each electrode combination, and the score of the similarity between the feature signal and the desired signal is performed based on corresponding weight coefficients to obtain the score corresponding to the each electrode combination.

In some optional embodiments, the weight coefficient corresponding to the similarity between the feature signal and the desired signal is greater than the weight coefficient of the signal strength of the feature signal, and the weight coefficient of the signal strength of the feature signal is greater than the weight coefficient of the pulse width of the feature signal.

The beneficial effect of the technical solution is as follows: The weight coefficient of the similarity between the feature signal and the desired signal, the weight coefficient of the signal strength of the feature signal, and the weight coefficient of the pulse width of the feature signal are in descending order, making a distinction and better meeting the requirements in practical application.

In some optional embodiments, the step of acquiring, based on one or more of the signal strength of the feature signal corresponding to the each electrode combination, the pulse width of the feature signal corresponding to the each electrode combination, or the similarity between the feature signal and the desired signal, the score corresponding to the each electrode combination includes acquiring, based on one or more of the signal strength of the feature signal corresponding to the each electrode combination, the pulse width of the feature signal corresponding to the each electrode combination, or the similarity between the feature signal and the desired signal, a score corresponding to an electrode combination when a feature signal corresponding to the electrode combination meets a preset condition. The preset condition includes one or more of the signal strength of the feature signal being greater than a preset signal strength or the pulse width of the feature signal satisfying a preset pulse width range.

In some optional embodiments, an acquisition process of the similarity between the feature signal and the desired signal is as follows: The feature signal and the desired signal are input into a similarity model to obtain the similarity between the feature signal and the desired signal. The similarity model is trained and obtained using a preset deep learning neural network.

In some optional embodiments, a training process of the similarity model is as follows: A first training signal and a second training signal are input into the preset deep learning neural network to obtain a predicted similarity between the first training signal and the second training signal; a predicted loss value is calculated and obtained based on the predicted similarity between the first training signal and the second training signal and an annotated similarity between the first training signal and the second training signal; and a parameter of the preset deep learning neural network is updated based on the predicted loss value to obtain the similarity model.

In a second aspect, the present application provides a system for recommending a stimulation electrode combination. The system includes an apparatus configured to receive potentials of a plurality of electrodes in a process of treatment delivery; an apparatus configured to calculate a difference between potentials of any two electrodes of the plurality of electrodes based on the potentials of the plurality of electrodes to obtain a voltage between the any two electrodes; an apparatus configured to acquire, based on the voltage between the any two electrodes within a preset time range, a feature signal corresponding to an electrode combination formed by the any two electrodes; and an apparatus configured to determine a recommended stimulation electrode combination from all electrode combinations based on a feature signal corresponding to each electrode combination.

In some optional embodiments, the apparatus configured to determine the recommended stimulation electrode combination from the all electrode combinations based on the feature signal corresponding to the each electrode combination includes an apparatus configured to acquire, based on one or more of a signal strength of the feature signal corresponding to the each electrode combination, a pulse width of the feature signal corresponding to the each electrode combination, or a similarity between the feature signal and a desired signal, a score corresponding to the each electrode combination and an apparatus configured to take an electrode combination with a highest score as the recommended stimulation electrode combination based on scores corresponding to the all electrode combinations.

In some optional embodiments, the apparatus configured to acquire, based on one or more of the signal strength of the feature signal corresponding to the each electrode combination, the pulse width of the feature signal corresponding to the each electrode combination, or the similarity between the feature signal and the desired signal, the score corresponding to the each electrode combination includes an apparatus configured to acquire a weight coefficient corresponding to the signal strength of the feature signal, a weight coefficient corresponding to the pulse width of the feature signal, and a weight coefficient corresponding to the similarity between the feature signal and the desired signal separately; an apparatus configured to acquire a score of the signal strength of the feature signal corresponding to the each electrode combination, a score of the pulse width of the feature signal corresponding to the each electrode combination, and a score of the similarity between the feature signal and the desired signal separately; and an apparatus configured to perform, based on corresponding weight coefficients, weighted summation on the score of the signal strength of the feature signal corresponding to the each electrode combination, the score of the pulse width of the feature signal corresponding to the each electrode combination, and the score of the similarity between the feature signal and the desired signal to obtain the score corresponding to the each electrode combination.

In some optional embodiments, the weight coefficient corresponding to the similarity between the feature signal and the desired signal is greater than the weight coefficient of the signal strength of the feature signal, and the weight coefficient of the signal strength of the feature signal is greater than the weight coefficient of the pulse width of the feature signal.

In some optional embodiments, the apparatus configured to acquire, based on one or more of the signal strength of the feature signal corresponding to the each electrode combination, the pulse width of the feature signal corresponding to the each electrode combination, or the similarity between the feature signal and the desired signal, the score corresponding to the each electrode combination includes an apparatus configured to acquire, based on one or more of the signal strength of the feature signal corresponding to the each electrode combination, the pulse width of the feature signal corresponding to the each electrode combination, or the similarity between the feature signal and the desired signal, a score corresponding to an electrode combination when a feature signal corresponding to the electrode combination meets a preset condition. The preset condition comprises one or more of the signal strength of the feature signal being greater than a preset signal strength or the pulse width of the feature signal satisfying a preset pulse width range.

In some optional embodiments, the apparatus configured to determine the recommended stimulation electrode combination from the all electrode combinations based on the feature signal corresponding to the each electrode combination also includes an apparatus configured to input the feature signal and the desired signal into a similarity model to obtain the similarity between the feature signal and the desired signal. The similarity model is trained and obtained using a preset deep learning neural network.

In some optional embodiments, a training process of the similarity model is as follows: A first training signal and a second training signal are input into the preset deep learning neural network to obtain a predicted similarity between the first training signal and the second training signal; a predicted loss value is calculated and obtained based on the predicted similarity between the first training signal and the second training signal and an annotated similarity between the first training signal and the second training signal; and a parameter of the preset deep learning neural network is updated based on the predicted loss value to obtain the similarity model.

In a third aspect, the present application provides a system for recommending a stimulation electrode combination. The system includes a plurality of electrodes, a treatment delivery circuit, a sensing circuit, and a controller. The plurality of electrodes are able to be positioned within a brain of a patient to deliver treatment to the patient or sense an electrical activity. The treatment delivery circuit is operably coupled to the plurality of electrodes to deliver the treatment to the patient. The sensing circuit is operably coupled to the plurality of electrodes to sense the electrical activity. The controller includes a processing circuit system operably coupled to the treatment delivery circuit and the sensing circuit. The controller is configured to control, through the treatment delivery circuit, one or more of the plurality of electrodes to deliver the treatment to the patient; sense potentials of the plurality of electrodes through the sensing circuit in a process of treatment delivery; calculate a difference between potentials of any two electrodes of the plurality of electrodes based on the potentials of the plurality of electrodes to obtain a voltage between the any two electrodes; acquire, based on the voltage between the any two electrodes within a preset time range, a feature signal corresponding to an electrode combination formed by the any two electrodes; and determine a recommended stimulation electrode combination from all electrode combinations based on a feature signal corresponding to each electrode combination.

In some optional embodiments, the step of determining the recommended stimulation electrode combination from the all electrode combinations based on the feature signal corresponding to the each electrode combination includes that a score corresponding to the each electrode combination is acquired based on one or more of a signal strength of the feature signal corresponding to the each electrode combination, a pulse width of the feature signal corresponding to the each electrode combination, or a similarity between the feature signal and a desired signal and that an electrode combination with a highest score is taken as the recommended stimulation electrode combination based on scores corresponding to the all electrode combinations.

In some optional embodiments, the step of acquiring, based on one or more of the signal strength of the feature signal corresponding to the each electrode combination, the pulse width of the feature signal corresponding to the each electrode combination, or the similarity between the feature signal and the desired signal, the score corresponding to the each electrode combination includes that a weight coefficient corresponding to the signal strength of the feature signal, a weight coefficient corresponding to the pulse width of the feature signal, and a weight coefficient corresponding to the similarity between the feature signal and the desired signal are configured separately, that a score of the signal strength of the feature signal corresponding to the each electrode combination, a score of the pulse width of the feature signal corresponding to the each electrode combination, and a score of the similarity between the feature signal and the desired signal are acquired separately, and that weighted summation on the score of the signal strength of the feature signal corresponding to the each electrode combination, the score of the pulse width of the feature signal corresponding to the each electrode combination, and the score of the similarity between the feature signal and the desired signal is performed based on corresponding weight coefficients to obtain the score corresponding to the each electrode combination.

In some optional embodiments, the weight coefficient corresponding to the similarity between the feature signal and the desired signal is greater than the weight coefficient of the signal strength of the feature signal, and the weight coefficient of the signal strength of the feature signal is greater than the weight coefficient of the pulse width of the feature signal.

In some optional embodiments, the step of acquiring, based on one or more of the signal strength of the feature signal corresponding to the each electrode combination, the pulse width of the feature signal corresponding to the each electrode combination, or the similarity between the feature signal and the desired signal, the score corresponding to the each electrode combination includes acquiring, based on one or more of the signal strength of the feature signal corresponding to the each electrode combination, the pulse width of the feature signal corresponding to the each electrode combination, or the similarity between the feature signal and the desired signal, a score corresponding to an electrode combination when a feature signal corresponding to the electrode combination meets a preset condition. The preset condition includes one or more of the signal strength of the feature signal being greater than a preset signal strength or the pulse width of the feature signal satisfying a preset pulse width range.

In some optional embodiments, an acquisition process of the similarity between the feature signal and the desired signal is as follows: The feature signal and the desired signal are input into a similarity model to obtain the similarity between the feature signal and the desired signal. The similarity model is trained and obtained using a preset deep learning neural network.

In some optional embodiments, a training process of the similarity model is as follows: A first training signal and a second training signal are input into the preset deep learning neural network to obtain a predicted similarity between the first training signal and the second training signal; a predicted loss value is calculated and obtained based on the predicted similarity between the first training signal and the second training signal and an annotated similarity between the first training signal and the second training signal; and a parameter of the preset deep learning neural network is updated based on the predicted loss value to obtain the similarity model.

In some optional embodiments, the electrical activity able to be sensed by the plurality of electrodes includes an electrical activity of delivering the treatment to the patient and a bioelectrical activity of the patient.

In some optional embodiments, the bioelectrical activity of the patient is an electrical activity of a single cell, an electrical activity of a nucleus, or an electrical activity of a part of a nucleus.

In a fourth aspect, the present application provides a computer-readable storage medium for storing a computer program. When executed by a processor, the computer program performs the steps in any preceding method for recommending a stimulation electrode combination.

The preceding description is only an overview of the technical solutions of the present application. In order that the technical means of the present application can be understood more clearly and implemented according to contents in the specification, preferred embodiments of the present application are described in detail below in conjunction with drawings.

### BRIEF DESCRIPTION OF DRAWINGS

The present application is described below in conjunction with drawings and embodiments.
FIG. 1 is a flowchart of a method for recommending a stimulation electrode combination according to embodiments of the present application.
FIG. 2 is a flowchart of determining a recommended stimulation electrode combination from all electrode combinations according to embodiments of the present application.
FIG. 3 is a flowchart of acquiring a score corresponding to each electrode combination according to embodiments of the present application.
FIG. 4 is a structural diagram of a system for recommending a stimulation electrode combination according to embodiments of the present application.
FIG. 5 is a structural diagram of an apparatus for determining a recommended stimulation electrode combination from all electrode combinations according to embodiments of the present application.
FIG. 6 is a structural diagram of an apparatus for acquiring a score corresponding to each electrode combination according to embodiments of the present application.
FIG. 7 is a structural diagram of a system for recommending a stimulation electrode combination according to embodiments of the present application.
FIG. 8 is a structural diagram of a program product according to embodiments of the present application.

### DETAILED DESCRIPTION

The present application will be further described in detail in conjunction with drawings and specific embodiments. It is to be noted that the embodiments or technical features described below may be arbitrarily combined to form a new embodiment on the premise of not conflicting each other. Specific details are set forth below to facilitate a thorough understanding of the present application. However, the present application may be implemented by other embodiments different from the embodiments described herein.

Therefore, the scope of the present application is subject to claims of the present application and is not limited by the embodiments described below.

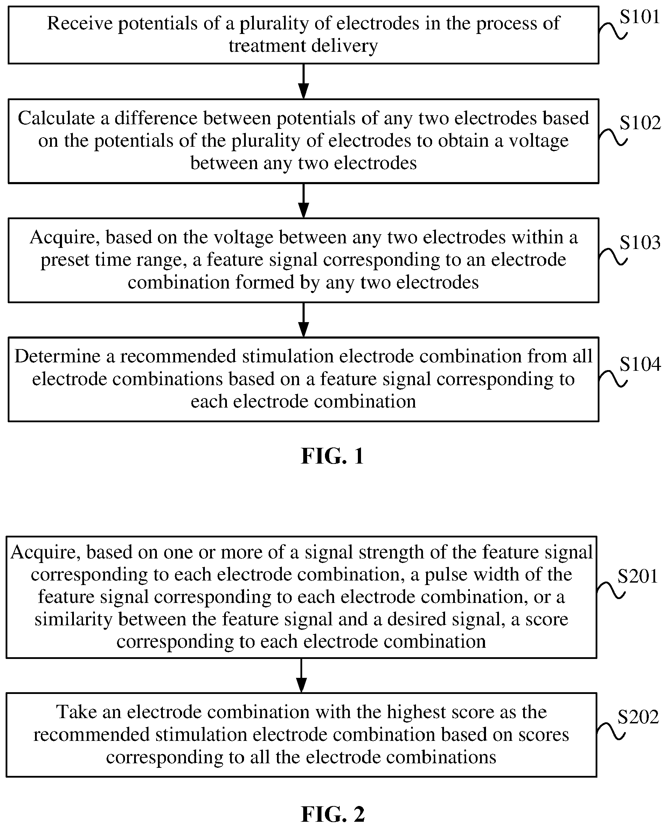

Referring to FIG. 1, embodiments of the present application provide a method for recommending a stimulation electrode combination. The method may include steps S 101 to S104.

In step S101, potentials of a plurality of electrodes are received in the process of treatment delivery.

In step S 102, a difference between potentials of any two electrodes is calculated based on the potentials of the plurality of electrodes to obtain a voltage between any two electrodes.

In step S 103, a feature signal corresponding to an electrode combination formed by any two electrodes is acquired based on the voltage between any two electrodes within a preset time range.

The preset time range may be a fixed time range or may be set automatically or manually according to different patient types. For example, the preset time range may be 30 s or 3 min. Alternatively, the preset time range may be set to 1 min for Parkinson's patients and 2 min for depression patients.

In step S104, a recommended stimulation electrode combination is determined from all electrode combinations based on a feature signal corresponding to each electrode combination.

In some embodiments, after the recommended stimulation electrode combination is determined from all the electrode combinations, the recommended stimulation electrode combination may be notified to a doctor or another medical worker in the form of an image, a video, text, or voice.

Accordingly, the potentials of the plurality of electrodes are sensed by the sensing circuit at the same time of treatment delivery so as to calculate the voltage between any two electrodes. A feature signal corresponding to an electrode combination formed by the two electrodes is acquired according to the voltage between any two electrodes within the preset time range. The recommended stimulation electrode combination is determined based on the feature signal. The manner of simultaneous stimulation and collection can accurately reflect the real-time state of a patient when receiving electrical stimulation, improving the accuracy of the stimulation electrode combination. The arrangement of acquiring the feature signal according to the voltage between any two electrodes and determining the recommended stimulation electrode combination can reduce the workload of the doctor, improve the working efficiency of the doctor, and further improve the accuracy of the doctor in selecting a stimulation electrode combination.

Referring to FIG. 2, in some embodiments, step S104 may include steps S201 to S202.

In step S201, a score corresponding to each electrode combination is acquired based on one or more of a signal strength of the feature signal corresponding to each electrode combination, a pulse width of the feature signal corresponding to each electrode combination, or a similarity between the feature signal and a desired signal.

In step S202, an electrode combination with the highest score is taken as the recommended stimulation electrode combination based on scores corresponding to all the electrode combinations.

For example, 45 electrode combinations are provided in total. The scores corresponding to all the electrode combinations from low to high are 23, 30, 80..., and 93. In this case, an electrode combination corresponding to score 93 is selected as the recommended stimulation electrode combination.

Accordingly, the score corresponding to each electrode combination is acquired based on one or more of the signal strength of the feature signal corresponding to each electrode combination, the pulse width of the feature signal corresponding to each electrode combination, or the similarity between the feature signal and the desired signal. The electrode combination with the highest score is taken as the recommended stimulation electrode combination. One or more of the signal strength, pulse width, or the similarity between the feature signal and the desired signal are taken as the basis of recommendation, meeting the actual requirements of treating the patient and enabling the recommended electrode combination to achieve a better treatment effect in practical application.

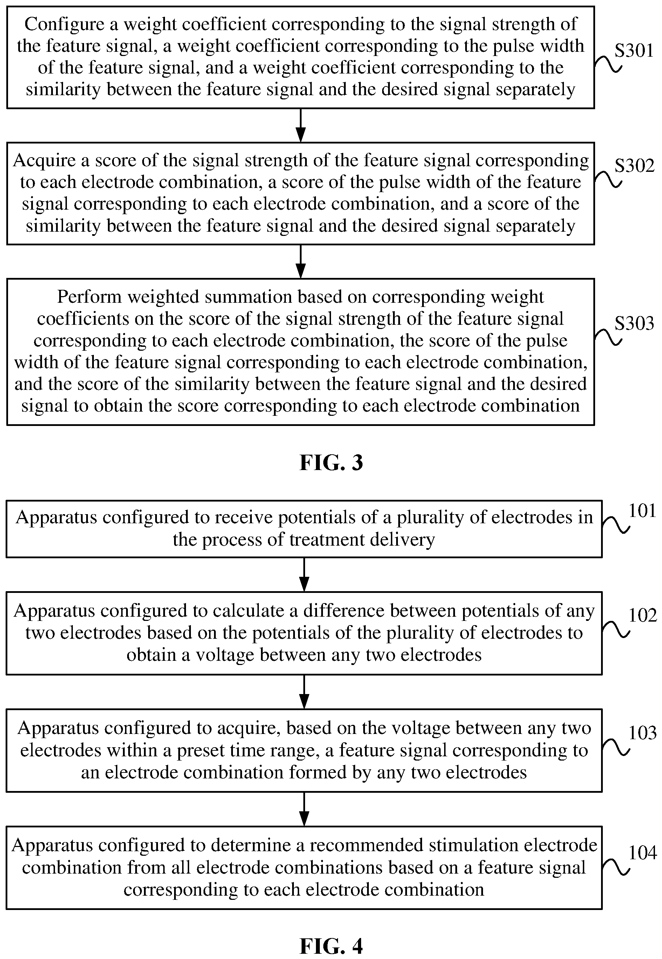

Referring to FIG. 3, in some embodiments, step S201 may include steps S301 to S303.

In step S301, a weight coefficient corresponding to the signal strength of the feature signal, a weight coefficient corresponding to the pulse width of the feature signal, and a weight coefficient corresponding to the similarity between the feature signal and the desired signal are configured separately.

Each weight coefficient may be any number between 0 and 100%. For example, weight coefficients may be configured as follows: The weight coefficient corresponding to the signal strength is 20%, the weight coefficient corresponding to the pulse width is 20%, and the weight coefficient corresponding to the similarity between the feature signal and the desired signal is 60%. In this configuration, priority is given to the effect of the similarity between the feature signal and the desired signal on the score. Alternatively, the weight coefficient corresponding to the signal strength is 0%, the weight coefficient corresponding to the pulse width is 0%, and the weight coefficient corresponding to the similarity between the feature signal and the desired signal is 100%. In this configuration, the effect of the similarity between the feature signal and the desired signal on the score is merely considered while the effect of the signal strength and pulse width on the score is considered comprehensively.

In step S302, a score of the signal strength of the feature signal corresponding to each electrode combination, a score of the pulse width of the feature signal corresponding to each electrode combination, and a score of the similarity between the feature signal and the desired signal are acquired separately.

Each score may be a percentile score. For example, the score of the signal strength may be a ratio of the signal strength to a standard signal strength multiplied by 100. The signal strength is 7 mv. The standard signal strength is 10 mv. In this case, the score of the signal strength is 70. The similarity between the feature signal and the desired signal may also be expressed in a percentile system. For example, the similarity between the feature signal and the desired signal is 80%; in this case, the score of the similarity between the feature signal and the desired signal is 80.

In step S303, weighted summation is performed based on corresponding weight coefficients on the score of the signal strength of the feature signal corresponding to each electrode combination, the score of the pulse width of the feature signal corresponding to each electrode combination, and the score of the similarity between the feature signal and the desired signal to obtain the score corresponding to each electrode combination.

Accordingly, the score of the signal strength of the feature signal, the score of the pulse width of the feature signal, and the score of the similarity between the feature signal and the desired signal are acquired separately. Then weighted summation is performed based on the configured weight coefficients to obtain the score corresponding to each electrode combination. The score integrating various factors is obtained by configuring the weight coefficients so that the score can better reflect the real situation of the patient. On this basis, the recommended electrode combination can meet the actual requirements of the patient more accurately.

In some embodiments, the weight coefficient corresponding to the similarity between the feature signal and the desired signal is greater than the weight coefficient of the signal strength of the feature signal, and the weight coefficient of the signal strength of the feature signal is greater than the weight coefficient of the pulse width of the feature signal.

Accordingly, the weight coefficient of the similarity between the feature signal and the desired signal, the weight coefficient of the signal strength of the feature signal, and the weight coefficient of the pulse width of the feature signal are in descending order, making a distinction and better meeting the requirements in practical application.

In some embodiments, step S201 may include step S401.

In step S401, a score corresponding to an electrode combination is acquired based on one or more of the signal strength of the feature signal corresponding to each electrode combination, the pulse width of the feature signal corresponding to each electrode combination, or the similarity between the feature signal and the desired signal when a feature signal corresponding to the electrode combination meets a preset condition. For example, the preset condition may be that the signal strength of the feature signal is greater than a preset signal strength, may be that the pulse width of the feature signal satisfies a preset pulse width range, or may be that the signal strength of the feature signal is greater than a preset signal strength and the pulse width of the feature signal satisfies a preset pulse width range.

Accordingly, when the feature signal corresponding to the electrode combination meets the preset condition, the score corresponding to the electrode combination can be acquired, avoiding a recommendation when the feature signal corresponding to the electrode combination does not meet the preset condition. It guarantees that the recommended electrode combination can meet the requirements of the patient.

In some embodiments, step S201 may include step S501.

In step S501, the feature signal and the desired signal are input into a similarity model to obtain the similarity between the feature signal and the desired signal. The similarity model is trained and obtained using a preset deep learning neural network.

Accordingly, the deep learning neural network is used to train and obtain the similarity model. Then the similarity model is used to obtain the similarity between the feature signal and the desired signal. The trained and obtained module is used for similarity acquisition, resulting in high efficiency and high accuracy.

In some embodiments, the training process of the similarity model may be as follows: A first training signal and a second training signal are input into the preset deep learning neural network to obtain a predicted similarity between the first training signal and the second training signal; a predicted loss value is calculated and obtained based on the predicted similarity between the first training signal and the second training signal and an annotated similarity between the first training signal and the second training signal; and a parameter of the preset deep learning neural network is updated based on the predicted loss value to obtain the similarity model.

The first training signal, the second training signal, and the annotated similarity may be acquired from a training database pre-stored in a storage medium. The first training signal is, for example, a feature signal acquired when any patient is treated. The second training signal is, for example, a feature signal corresponding to a patient with better treatment effect and screened from feature signals collected during the treatment of all patients. The annotated similarity may be obtained by manual annotation. In the training process, supervised learning or semi-supervised learning may be adopted.

Accordingly, a method for training a similarity model is provided. The similarity model uses the first training signal and the second training signal for training so that the calculation is fast and has an accurate result when the similarity module is applied to calculating the similarity of the feature signal and the desired signal.

Referring to FIG. 4, embodiments of the present application provide a system for recommending a stimulation electrode combination. The specific implementation of the system is consistent with the implementation and technical effects in embodiments of the preceding method for recommending a stimulation electrode combination. Part of the content is not repeated. The system for recommending a stimulation electrode combination includes an apparatus 101, an apparatus 102, an apparatus 103, and an apparatus 104. The apparatus 101 is configured to receive potentials of a plurality of electrodes in the process of treatment delivery. The apparatus 102 is configured to calculate a difference between potentials of any two electrodes based on the potentials of the plurality of electrodes to obtain a voltage between any two electrodes. The apparatus 103 is configured to acquire, based on the voltage between any two electrodes within a preset time range, a feature signal corresponding to an electrode combination formed by any two electrodes. The apparatus 104 is configured to determine a recommended stimulation electrode combination from all electrode combinations based on a feature signal corresponding to each electrode combination.

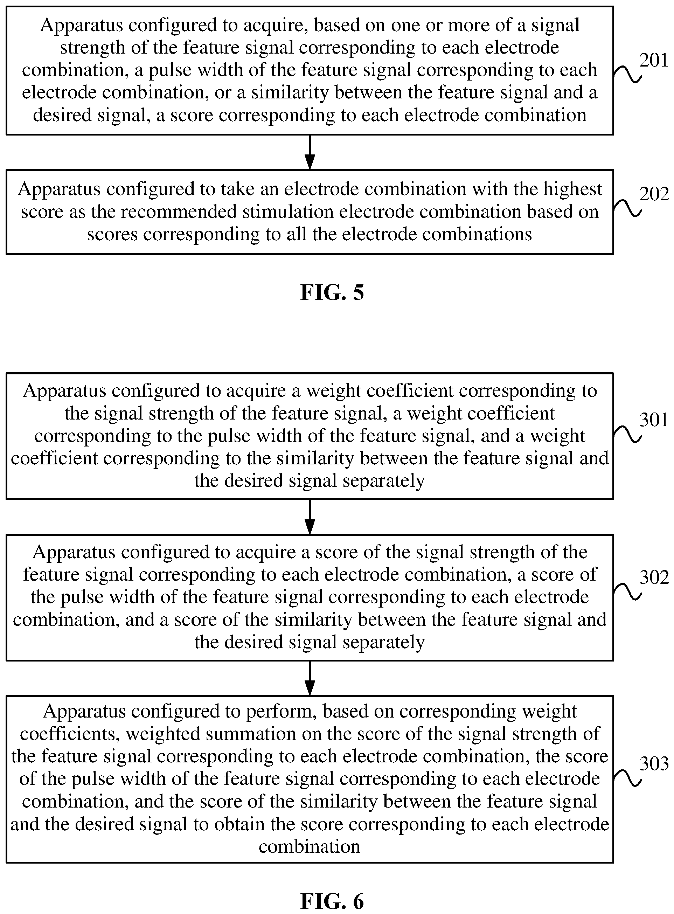

Referring to FIG. 5, in some embodiments, the apparatus 104 configured to determine the recommended stimulation electrode combination from all the electrode combinations based on the feature signal corresponding to each electrode combination may include an apparatus 201 and an apparatus 202. The apparatus 201 is configured to acquire, based on one or more of a signal strength of the feature signal corresponding to each electrode combination, a pulse width of the feature signal corresponding to each electrode combination, or a similarity between the feature signal and a desired signal, a score corresponding to each electrode combination. The apparatus 202 is configured to take an electrode combination with the highest score as the recommended stimulation electrode combination based on scores corresponding to all the electrode combinations.

Referring to FIG. 6, in some embodiments, the apparatus 201 configured to acquire, based on one or more of the signal strength of the feature signal corresponding to each electrode combination, the pulse width of the feature signal corresponding to each electrode combination, or the similarity between the feature signal and the desired signal, the score corresponding to each electrode combination may include an apparatus 301, an apparatus 302, and an apparatus 303. The apparatus 301 is configured to acquire a weight coefficient corresponding to the signal strength of the feature signal, a weight coefficient corresponding to the pulse width of the feature signal, and a weight coefficient corresponding to the similarity between the feature signal and the desired signal separately. The apparatus 302 is configured to acquire a score of the signal strength of the feature signal corresponding to each electrode combination, a score of the pulse width of the feature signal corresponding to each electrode combination, and a score of the similarity between the feature signal and the desired signal separately. The apparatus 303 is configured to perform, based on corresponding weight coefficients, weighted summation on the score of the signal strength of the feature signal corresponding to each electrode combination, the score of the pulse width of the feature signal corresponding to each electrode combination, and the score of the similarity between the feature signal and the desired signal to obtain the score corresponding to each electrode combination.

In some embodiments, the weight coefficient corresponding to the similarity between the feature signal and the desired signal is greater than the weight coefficient of the signal strength of the feature signal, and the weight coefficient of the signal strength of the feature signal is greater than the weight coefficient of the pulse width of the feature signal.

In some embodiments, the apparatus 201 configured to acquire, based on one or more of the signal strength of the feature signal corresponding to each electrode combination, the pulse width of the feature signal corresponding to each electrode combination, or the similarity between the feature signal and the desired signal, the score corresponding to each electrode combination may include an apparatus 401 configured to acquire, based on one or more of the signal strength of the feature signal corresponding to each electrode combination, the pulse width of the feature signal corresponding to each electrode combination, or the similarity between the feature signal and the desired signal, a score corresponding to an electrode combination when a feature signal corresponding to the electrode combination meets a preset condition. The preset condition includes one or more of the signal strength of the feature signal being greater than a preset signal strength or the pulse width of the feature signal satisfying a preset pulse width range.

In some embodiments, the apparatus 104 configured to determine the recommended stimulation electrode combination from all the electrode combinations based on the feature signal corresponding to each electrode combination may also include an apparatus 501 configured to input the feature signal and the desired signal into a similarity model to obtain the similarity between the feature signal and the desired signal. The similarity model is trained and obtained using a preset deep learning neural network.

In some embodiments, the training process of the similarity model may be as follows: A first training signal and a second training signal are input into the preset deep learning neural network to obtain a predicted similarity between the first training signal and the second training signal; a predicted loss value is calculated and obtained based on the predicted similarity between the first training signal and the second training signal and an annotated similarity between the first training signal and the second training signal; and a parameter of the preset deep learning neural network is updated based on the predicted loss value to obtain the similarity model.

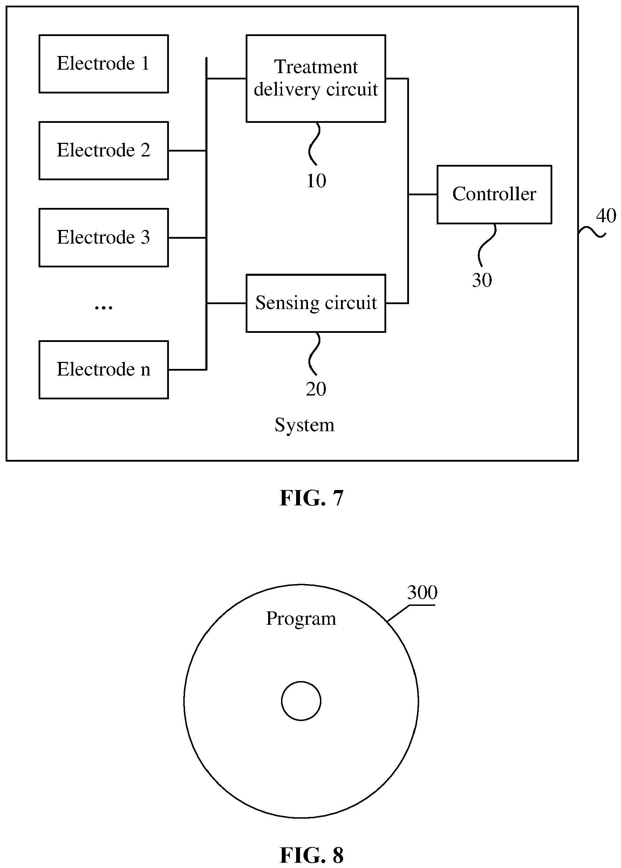

Referring to FIG. 7, embodiments of the present application provide a system 40 for recommending a stimulation electrode combination. The specific implementation of the system is consistent with the implementation and technical effects in embodiments of the preceding method for recommending a stimulation electrode combination. Part of the content is not repeated. The system 40 for recommending a stimulation electrode combination may include a plurality of electrodes, a treatment delivery circuit 10, a sensing circuit 20, and a controller 30. The plurality of electrodes are able to be positioned within the brain of a patient to deliver treatment to the patient or sense an electrical activity. The treatment delivery circuit 10 is operably coupled to the plurality of electrodes to deliver the treatment to the patient. The sensing circuit 20 is operably coupled to the plurality of electrodes to sense the electrical activity. The controller 30 includes a processing circuit system operably coupled to the treatment delivery circuit 10 and the sensing circuit 20. The controller 30 is configured to control, through the treatment delivery circuit 10, one or more of the plurality of electrodes to deliver the treatment to the patient; sense potentials of the plurality of electrodes through the sensing circuit 20 in the process of treatment delivery; calculate a difference between potentials of any two electrodes based on the potentials of the plurality of electrodes to obtain a voltage between any two electrodes; acquire, based on the voltage between any two electrodes within a preset time range, a feature signal corresponding to an electrode combination formed by any two electrodes; and determine a recommended stimulation electrode combination from all electrode combinations based on a feature signal corresponding to each electrode combination.

The treatment delivery circuit 10 may include a pulse generator and an extension lead connected to the pulse generator. One end of the extension lead is connected to the pulse generator, and the other end of the extension lead is connected to an electrode. Therefore, a pulse generated by the pulse generator is transmitted to the electrode. The electrode is used for delivering the treatment to the patient. The pulse generator may be coupled to the controller 30.

The controller 30 may include a memory and a processor. The size, shape, and position of the controller 30 are not limited in the present application. The control function of the controller 30 may be implemented by an MPU, an MCU, a DSP, an FPGA, or any combination thereof. With the adoption of an integrated chip, the wireless upgrade of an embedded program is supported.

When sensing the electrical activity, the system 40 in the present application controls, through the treatment delivery circuit 10, one or more of the plurality of electrodes to deliver the treatment to the patient, senses the potentials of the plurality of electrodes through the sensing circuit 20, and calculates the difference between the potentials of any two electrodes based on the potentials of the plurality of electrodes to obtain the voltage between any two electrodes.

Since the sensing of the electrical activity is synchronized with the treatment delivery to the patient, the measured voltage can reflect the brain condition of the patient when receiving the treatment delivery, which has a great reference value for the follow-up treatment of the patient.

In some embodiments, the step of determining the recommended stimulation electrode combination from all the electrode combinations based on the feature signal corresponding to each electrode combination may include that a score corresponding to each electrode combination is acquired based on one or more of a signal strength of the feature signal corresponding to each electrode combination, a pulse width of the feature signal corresponding to each electrode combination, or a similarity between the feature signal and a desired signal and that an electrode combination with the highest score is taken as the recommended stimulation electrode combination based on scores corresponding to all the electrode combinations.

In some embodiments, the step of acquiring, based on one or more of the signal strength of the feature signal corresponding to each electrode combination, the pulse width of the feature signal corresponding to each electrode combination, or the similarity between the feature signal and a desired signal, the score corresponding to each electrode combination may include that a weight coefficient corresponding to the signal strength of the feature signal, a weight coefficient corresponding to the pulse width of the feature signal, and a weight coefficient corresponding to the similarity between the feature signal and the desired signal are acquired separately, that a score of the signal strength of the feature signal corresponding to each electrode combination, a score of the pulse width of the feature signal corresponding to each electrode combination, and a score of the similarity between the feature signal and the desired signal are acquired separately, and weighted summation on the score of the signal strength of the feature signal corresponding to each electrode combination, the score of the pulse width of the feature signal corresponding to each electrode combination, and the score of the similarity between the feature signal and the desired signal is performed based on corresponding weight coefficients to obtain the score corresponding to each electrode combination.

In some embodiments, the weight coefficient corresponding to the similarity between the feature signal and the desired signal is greater than the weight coefficient of the signal strength of the feature signal, and the weight coefficient of the signal strength of the feature signal is greater than the weight coefficient of the pulse width of the feature signal.

In some embodiments, the step of acquiring, based on one or more of the signal strength of the feature signal corresponding to each electrode combination, the pulse width of the feature signal corresponding to each electrode combination, or the similarity between the feature signal and the desired signal, the score corresponding to each electrode combination may include acquiring, based on one or more of the signal strength of the feature signal corresponding to each electrode combination, the pulse width of the feature signal corresponding to each electrode combination, or the similarity between the feature signal and the desired signal, a score corresponding to an electrode combination when a feature signal corresponding to the electrode combination meets a preset condition. The preset condition includes one or more of the signal strength of the feature signal being greater than a preset signal strength or the pulse width of the feature signal satisfying a preset pulse width range.

In some embodiments, the acquisition process of the similarity between the feature signal and the desired signal is as follows: The feature signal and the desired signal are input into a similarity model to obtain the similarity between the feature signal and the desired signal. The similarity model is trained and obtained using a preset deep learning neural network.

In some embodiments, the training process of the similarity model may be as follows: A first training signal and a second training signal are input into the preset deep learning neural network to obtain a predicted similarity between the first training signal and the second training signal; a predicted loss value is calculated and obtained based on the predicted similarity between the first training signal and the second training signal and an annotated similarity between the first training signal and the second training signal; and a parameter of the preset deep learning neural network is updated based on the predicted loss value to obtain the similarity model.

In some embodiments, the electrical activity able to be sensed by the plurality of electrodes includes an electrical activity of delivering the treatment to the patient and a bioelectrical activity of the patient.

In general, an electrode required for collecting a single cell electrical signal is different from an electrode required for collecting a local field potential. A single cell requires a small electrode or a microelectrode. The signal collected out of the single cell has high amplitude and frequency. The local field potential requires a normal electrode or a macro electrode, and the collected signal has low amplitude and low frequency. The size of a normal electrode is, for example, 6 square millimeters.

The system in the present application may use the plurality of electrodes to sense an electrical activity of a single cell, an electrical activity of a nucleus, or an electrical activity of a part of a nucleus.

In some embodiments, the bioelectrical activity of the patient is an electrical activity of a single cell, an electrical activity of a nucleus, or an electrical activity of a part of a nucleus.

Embodiments of the present application further provide a computer-readable storage medium configured to store a computer program. When executed, the computer program performs steps of a single cell discharge collection method in embodiments of the present application. The specific implementation of the computer program is consistent with the implementation and technical effects in embodiments of the preceding single cell discharge collection. Part of the content is not repeated

FIG. 8 illustrates a program product 300 used for performing the preceding single cell discharge collection method according to this embodiment. The program product 300 may use a portable compact disk read-only memory (CD-ROM) and includes program code. The program products 300 may run on a terminal device, such as a personal computer. However, the program product 300 of the present invention is not limited herein. In the present application, the readable storage medium may be any tangible medium including or storing a program. The program may be used by or used in conjunction with an instruction execution system, apparatus or device. The program product 300 may use any combination of one or more readable media. A readable medium may be a readable signal medium or a readable storage medium. The readable storage medium may be, but is not limited to, an electrical, magnetic, optical, electromagnetic, infrared or semiconductor system, apparatus or device, or any combination thereof. More specific examples of the readable storage medium include (non-exhaustive list): an electrical connection having one or more wires, a portable computer magnetic disk, a hard disk, a random access memory (RAM), a read only memory (ROM), an erasable programmable read only memory (EPROM or flash memory), an optical fiber, a portable compact disk read only memory (CD-ROM), an optical memory device, a magnetic memory device, or any suitable combination thereof.

The computer-readable storage medium may include a data signal propagated in a baseband or as part of a carrier. Readable program codes are carried in the data signal. The data signal propagated in this manner may be in multiple forms and includes, but is not limited to, an electromagnetic signal, an optical signal or any suitable combination thereof. The readable storage medium may be any readable medium. The readable medium may send, propagate, or transmit a program used by or used in conjunction with an instruction execution system, apparatus, or device. Program code contained in the readable storage medium may be transmitted via any suitable medium. The medium includes, but is not limited to, the wireless, a wire, an optical cable, radio frequency (RF), or the like, or any suitable combination thereof. Program codes for performing the operations of the present application may be written in one or more programming languages or a combination thereof, the programming languages including object-oriented programming languages such as Java and C++ and further including conventional procedural programming languages such as C programming language or similar programming languages. The program codes may be executed entirely on a user computing device, executed partly on a user device, executed as a stand-alone software package, executed partly on a user computing device and partly on a remote computing device, or executed entirely on a remote computing device or a server. In the case where the remote computing device is involved, the remote computing device may be connected to the user computing device via any type of network including a local area network (LAN) or a wide area network (WAN) or may be connected to an external computing device (for example, via the Internet provided by an Internet service provider).

The present application is elaborated from viewpoints including use purpose, efficiency, progress, and novelty, which has met the functional enhancement and use requirements emphasized in patent law. The preceding specification and drawings of the specification are only preferred embodiments of the present application and are not intended to limit the scope of the present application. Therefore, any similar or identical structures, apparatuses, and features of the present application, that is, any equivalent replacements or modifications made according to the scope of the present application, shall fall within the scope of the present application.

## Claims

1. A method for recommending a stimulation electrode combination, comprising:
receiving potentials of a plurality of electrodes in a process of treatment delivery;
calculating a difference between potentials of any two electrodes of the plurality of electrodes based on the potentials of the plurality of electrodes to obtain a voltage between the any two electrodes;
acquiring, based on the voltage between the any two electrodes within a preset time range, a feature signal corresponding to an electrode combination formed by the any two electrodes; and
determining a recommended stimulation electrode combination from all electrode combinations based on a feature signal corresponding to each electrode combination.

2. The method according to claim 1, wherein determining the recommended stimulation electrode combination from the all electrode combinations based on the feature signal corresponding to the each electrode combination comprises:
acquiring, based on one or more of a signal strength of the feature signal corresponding to the each electrode combination, a pulse width of the feature signal corresponding to the each electrode combination, or a similarity between the feature signal and a desired signal, a score corresponding to the each electrode combination; and
taking an electrode combination with a highest score as the recommended stimulation electrode combination based on scores corresponding to the all electrode combinations.

3. The method according to claim 2, wherein acquiring, based on one or more of the signal strength of the feature signal corresponding to the each electrode combination, the pulse width of the feature signal corresponding to the each electrode combination, or the similarity between the feature signal and the desired signal, the score corresponding to the each electrode combination comprises:
configuring a weight coefficient corresponding to the signal strength of the feature signal, a weight coefficient corresponding to the pulse width of the feature signal, and a weight coefficient corresponding to the similarity between the feature signal and the desired signal separately;
acquiring a score of the signal strength of the feature signal corresponding to the each electrode combination, a score of the pulse width of the feature signal corresponding to the each electrode combination, and a score of the similarity between the feature signal and the desired signal separately; and
performing, based on corresponding weight coefficients, weighted summation on the score of the signal strength of the feature signal corresponding to the each electrode combination, the score of the pulse width of the feature signal corresponding to the each electrode combination, and the score of the similarity between the feature signal and the desired signal to obtain the score corresponding to the each electrode combination.

4. The method according to claim 3, wherein the weight coefficient corresponding to the similarity between the feature signal and the desired signal is greater than the weight coefficient of the signal strength of the feature signal, and the weight coefficient of the signal strength of the feature signal is greater than the weight coefficient of the pulse width of the feature signal.

5. A system for recommending a stimulation electrode combination, comprising:
an apparatus configured to receive potentials of a plurality of electrodes in a process of treatment delivery;
an apparatus configured to calculate a difference between potentials of any two electrodes of the plurality of electrodes based on the potentials of the plurality of electrodes to obtain a voltage between the any two electrodes;
an apparatus configured to acquire, based on the voltage between the any two electrodes within a preset time range, a feature signal corresponding to an electrode combination formed by the any two electrodes; and
an apparatus configured to determine a recommended stimulation electrode combination from all electrode combinations based on a feature signal corresponding to each electrode combination.

6. The system according to claim 5, wherein the apparatus configured to determine the recommended stimulation electrode combination from the all electrode combinations based on the feature signal corresponding to the each electrode combination comprises:
an apparatus configured to acquire, based on one or more of a signal strength of the feature signal corresponding to the each electrode combination, a pulse width of the feature signal corresponding to the each electrode combination, or a similarity between the feature signal and a desired signal, a score corresponding to the each electrode combination; and
an apparatus configured to take an electrode combination with a highest score as the recommended stimulation electrode combination based on scores corresponding to the all electrode combinations.

7. The system according to claim 6, wherein the apparatus configured to acquire, based on one or more of the signal strength of the feature signal corresponding to the each electrode combination, the pulse width of the feature signal corresponding to the each electrode combination, or the similarity between the feature signal and the desired signal, the score corresponding to the each electrode combination comprises:
an apparatus configured to acquire a weight coefficient corresponding to the signal strength of the feature signal, a weight coefficient corresponding to the pulse width of the feature signal, and a weight coefficient corresponding to the similarity between the feature signal and the desired signal separately;
an apparatus configured to acquire a score of the signal strength of the feature signal corresponding to the each electrode combination, a score of the pulse width of the feature signal corresponding to the each electrode combination, and a score of the similarity between the feature signal and the desired signal separately; and
an apparatus configured to perform, based on corresponding weight coefficients, weighted summation on the score of the signal strength of the feature signal corresponding to the each electrode combination, the score of the pulse width of the feature signal corresponding to the each electrode combination, and the score of the similarity between the feature signal and the desired signal to obtain the score corresponding to the each electrode combination.

8. The system according to claim 7, wherein the weight coefficient corresponding to the similarity between the feature signal and the desired signal is greater than the weight coefficient of the signal strength of the feature signal, and the weight coefficient of the signal strength of the feature signal is greater than the weight coefficient of the pulse width of the feature signal.

9. The system according to claim 6, wherein the apparatus configured to acquire, based on one or more of the signal strength of the feature signal corresponding to the each electrode combination, the pulse width of the feature signal corresponding to the each electrode combination, or the similarity between the feature signal and the desired signal, the score corresponding to the each electrode combination comprises:
an apparatus configured to acquire, based on one or more of the signal strength of the feature signal corresponding to the each electrode combination, the pulse width of the feature signal corresponding to the each electrode combination, or the similarity between the feature signal and the desired signal, a score corresponding to an electrode combination when a feature signal corresponding to the electrode combination meets a preset condition, wherein the preset condition comprises one or more of the signal strength of the feature signal being greater than a preset signal strength or the pulse width of the feature signal satisfying a preset pulse width range.

10. The system according to claim 6, wherein an acquisition process of the similarity between the feature signal and the desired signal is as follows:
inputting the feature signal and the desired signal into a similarity model to obtain the similarity between the feature signal and the desired signal, wherein the similarity model is trained and obtained using a preset deep learning neural network.

11. The system according to claim 10, wherein a training process of the similarity model is as follows:
inputting a first training signal and a second training signal into the preset deep learning neural network to obtain a predicted similarity between the first training signal and the second training signal;
calculating and obtaining a predicted loss value based on the predicted similarity between the first training signal and the second training signal and an annotated similarity between the first training signal and the second training signal; and
updating a parameter of the preset deep learning neural network based on the predicted loss value to obtain the similarity model.

12. A system for recommending a stimulation electrode combination, comprising:
a plurality of electrodes able to be positioned within a brain of a patient to deliver treatment to the patient or sense an electrical activity;
a treatment delivery circuit operably coupled to the plurality of electrodes to deliver the treatment to the patient;
a sensing circuit operably coupled to the plurality of electrodes to sense the electrical activity; and
a controller comprising a processing circuit system operably coupled to the treatment delivery circuit and the sensing circuit, wherein the controller is configured to:
control, through the treatment delivery circuit, one or more of the plurality of electrodes to deliver the treatment to the patient;
sense potentials of the plurality of electrodes through the sensing circuit in a process of treatment delivery;
calculate a difference between potentials of any two electrodes of the plurality of electrodes based on the potentials of the plurality of electrodes to obtain a voltage between the any two electrodes;
acquire, based on the voltage between the any two electrodes within a preset time range, a feature signal corresponding to an electrode combination formed by the any two electrodes; and
determine a recommended stimulation electrode combination from all electrode combinations based on a feature signal corresponding to each electrode combination.

13. The system according to claim 12, wherein determining the recommended stimulation electrode combination from the all electrode combinations based on the feature signal corresponding to the each electrode combination comprises:
acquiring, based on one or more of a signal strength of the feature signal corresponding to the each electrode combination, a pulse width of the feature signal corresponding to the each electrode combination, or a similarity between the feature signal and a desired signal, a score corresponding to the each electrode combination; and
taking an electrode combination with a highest score as the recommended stimulation electrode combination based on scores corresponding to the all electrode combinations.

14. The system according to claim 13, wherein acquiring, based on one or more of the signal strength of the feature signal corresponding to the each electrode combination, the pulse width of the feature signal corresponding to the each electrode combination, or the similarity between the feature signal and the desired signal, the score corresponding to the each electrode combination comprises:
configuring a weight coefficient corresponding to the signal strength of the feature signal, a weight coefficient corresponding to the pulse width of the feature signal, and a weight coefficient corresponding to the similarity between the feature signal and the desired signal separately;
acquiring a score of the signal strength of the feature signal corresponding to the each electrode combination, a score of the pulse width of the feature signal corresponding to the each electrode combination, and a score of the similarity between the feature signal and the desired signal separately; and
performing, based on corresponding weight coefficients, weighted summation on the score of the signal strength of the feature signal corresponding to the each electrode combination, the score of the pulse width of the feature signal corresponding to the each electrode combination, and the score of the similarity between the feature signal and the desired signal to obtain the score corresponding to the each electrode combination.

15. The system according to claim 14, wherein the weight coefficient corresponding to the similarity between the feature signal and the desired signal is greater than the weight coefficient of the signal strength of the feature signal, and the weight coefficient of the signal strength of the feature signal is greater than the weight coefficient of the pulse width of the feature signal.

16. The system according to claim 13, wherein acquiring, based on one or more of the signal strength of the feature signal corresponding to the each electrode combination, the pulse width of the feature signal corresponding to the each electrode combination, or the similarity between the feature signal and the desired signal, the score corresponding to the each electrode combination comprises:
acquiring, based on one or more of the signal strength of the feature signal corresponding to the each electrode combination, the pulse width of the feature signal corresponding to the each electrode combination, or the similarity between the feature signal and the desired signal, a score corresponding to an electrode combination when a feature signal corresponding to the electrode combination meets a preset condition, wherein the preset condition comprises one or more of the signal strength of the feature signal being greater than a preset signal strength or the pulse width of the feature signal satisfying a preset pulse width range.

17. The system according to claim 13, wherein an acquisition process of the similarity between the feature signal and the desired signal is as follows:
inputting the feature signal and the desired signal into a similarity model to obtain the similarity between the feature signal and the desired signal, wherein the similarity model is trained and obtained using a preset deep learning neural network.

18. The system according to claim 17, wherein a training process of the similarity model is as follows:
inputting a first training signal and a second training signal into the preset deep learning neural network to obtain a predicted similarity between the first training signal and the second training signal;
calculating and obtaining a predicted loss value based on the predicted similarity between the first training signal and the second training signal and an annotated similarity between the first training signal and the second training signal; and
updating a parameter of the preset deep learning neural network based on the predicted loss value to obtain the similarity model.

19. The system according to claim 12, wherein the electrical activity able to be sensed by the plurality of electrodes comprises an electrical activity of delivering the treatment to the patient and a bioelectrical activity of the patient.

20. The system according to claim 19, wherein the bioelectrical activity of the patient is an electrical activity of a single cell, an electrical activity of a nucleus, or an electrical activity of a part of a nucleus.
